(19) Europäisches Patentamt
European Patent Office
Office européen des brevets

(11) Publication number: 0 180 719
B1

(12) # EUROPEAN PATENT SPECIFICATION

(45) Date of publication of patent specification: 21.03.90

(51) Int. Cl.$^5$: C 07 C 29/32

(21) Application number: 85109213.0

(22) Date of filing: 23.07.85

(54) **The preparation of ethanol and higher alcohols from lower carbon number alcohols.**

(30) Priority: 05.11.84 US 668598

(43) Date of publication of application:
14.05.86 Bulletin 86/20

(45) Publication of the grant of the patent:
21.03.90 Bulletin 90/12

(84) Designated Contracting States:
BE DE FR GB IT NL SE

(56) References cited:
WO-A-84/03696
GB-A-2 088 870
US-A-4 272 410

(73) Proprietor: THE DOW CHEMICAL COMPANY
2030 Dow Center Abbott Road P.O. Box 1967
Midland Michigan 48640-1967 (US)

(72) Inventor: Quarderer, George J.
4309 Moorland Drive
Midland, MI 48640 (US)
Inventor: Cochran, Gene A.
1403 Dilloway Drive
Midland, MI 48640 (US)
Inventor: Stevens, Rex R.
4311 Andre
Midland, MI 48640 (US)
Inventor: Murchison, Craig B.
606 West Meadowbrook Drive
Midland, MI 48640 (US)

(74) Representative: Casalonga, Axel et al
BUREAU D.A. CASALONGA - JOSSE
Morassistrasse 8
D-8000 München 5 (DE)

EP 0 180 719 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns a process for making ethanol and higher alcohols from alcohols with lower carbon numbers using hydrogen, carbon monoxide and a heterogeneous catalyst.

There are several significant processes for making mixed alcohols from synthesis gas or $H_2/CO$. For example, British patent publication 2,083,469 discloses such a process wherein the catalyst is based on chromium, zinc and at least one alkali metal. While the patent claims that the mixed alcohols may contain from 35 to 75 weight percent methanol, the examples given vary from 43 to 69 weight percent methanol.

PCT application 84.00405, filed March 16, 1984, (published September 27, 1984, as 84/03696) discloses a process for making mixed alcohols from synthesis gas using a molybdenum-based catalyst.

Substantial methanol may be coproduced with the higher alcohols in these prior art processes. The coproduction of methanol may be undesirable for a number of reasons. Also for economic reasons, the conversion of methanol to higher alcohols, especially ethanol, may be desired.

The major use for the synthesized mixed alcohols is as a fuel additive in gasoline engines. Methanol is an undesirable component for a number of reasons. When methanol is blended in hydrocarbon gasolines, methanol is said (a) to increase evaporative emissions and (b) to be liable to phase separation which in turn may lead to (i) corrosion of fuel systems and engine components and (ii) possible inferior drivability. While these deficiencies may be in dispute, it is at least desirable to minimize or at least reduce coproduced methanol.

One possible remedy is to make a lower percentage of methanol in the mixed alcohols. Another possible remedy is to lower the percentage of methanol in the mixed alcohols through catalyst selection. It would be desirable to make a lower methanol to higher alcohols ratio at a high productivity and without lowering the activity or selectivity of the catalyst to mixed alcohols.

This invention concerns making a $C_2$—$C_6$ alcohol from a lower carbon number alcohol. Preferably making a mixed alcohols stream with a lower methanol to higher alcohols ratio. More preferably making a mixed alcohol stream with less than 50 weight percent methanol while retaining high activity and selectivity to mixed alcohols. Clearly possible is conversion of lower value methanol into higher valued higher alcohols.

The present process enables higher alcohols to be prepared under conditions where the number of carbon atoms in the product alcohols are increased compared with the number of carbon atoms in the starting alcohols which comprises reacting:

(1) hydrogen;
(2) carbon monoxide;
(3) a heterogenous catalyst having (a) a first component of molybdenum, tungsten, or a mixture thereof in free or combinded form; and (b) a second component of an alkali or alkaline earth element, or a mixture thereof in free or combined form; and
(4) one or more lower alcohols.

It is a feature of the present process that methanol or another lower alcohol is combined with the $H_2/CO$ feed. One advantage of the present process is that the lower value methanol or other alcohols are apparently converted to a higher homologue. The present process unexpectedly converts as much as 40 weight percent (or higher) of carbon monoxide to alcohols with a decrease in methanol to higher alcohols ratio. The process appears to be a homologation reaction; however, the exact mechanism is uncertain.

The hydrogen and carbon monoxide required for this process may be obtained by methods known in the art. Examples are gasification of hydrocarbonaceous materials such as coal, high specific gravity oil, or natural gas; as a by-product of partial combustion cracking of hydrocarbons; by steam reforming of liquid or gaseous hydrocarbons; through the water-gas shift reaction; or some combination of these. The two components may also be generated separately and combined for the subject reaction. The molar ratio of hydrogen to carbon monoxide in the feed gas which contacts the catalyst ranges generally from about 0.25:1 to about 100:1, preferably from about 0.5:1 to about 5:1, and more preferably from about 0.7:1 to about 3:1. A most preferred range is from about 0.7:1 to about 1.5:1.

The one or more lower alcohols are preferably $C_1$—$C_6$ alcohols, more preferably $C_1$—$C_5$ alcohols. Exemplary alcohols include: aliphatic alcohols such as methanol, ethanol, 1-propanol, 2-propanol, n-butanol, 2-butanol, 2-methyl-2-propanol, and higher homologues; dihydroxy alcohols such as ethylene glycol, propylene glycol and 1,4-dihydroxybutane; trihydroxy alcohols such as glycerine; cycloaliphatic alcohols such as cyclohexanol and substituted cyclohexanols; and phenols and substituted phenols. The lower alcohols may be substituted. Preferably, the substituents are inert, meaning the substituent is not altered under the conditions experienced by the lower alcohol during the reaction.

Preferred lower alcohols are the $C_1$—$C_3$ alcohols. Methanol is most preferred because its higher homologues are substantially more valuable. A particularly preferred lower alcohol is one formed by fractionating the mixed alcohol product of the process to remove the methanol which is then recycled back into the feed. One may substantially reduce the methanol content of the mixed alcohols using this scheme or may recycle the methanol to extinction. Alternatively, one may bring in methanol from outside the process to add to the feed. This may not decrease the ratio of methanol to higher alcohols in the product but instead may act to upgrade the purchased methanol feed.

The first component of the catalyst preferably consists essentially of molybdenum, tungsten or a

mixture thereof in free or combined form. Molybdenum is preferred.

The molybdenum or tungsten may be present in the catalyst in "free or combined form" which means that it may be present as a metal, an alloy or a compound of the element. Representative compounds include the sulfides, carbides, oxides, halides, nitrides, borides, salicylides, oxyhalides, carboxylates such as acetates, acetylacetonates, oxalates, carbonyls and the like. Representative compounds also include the elements in anionic form such as molybdates, phosphomolybdates, tungstates, phosphotungstates, and the like and include the alkali, alkaline earth, rare earth and actinide series of salts of these anions. The sulfides, carbonyls, carbides and oxides are preferred, with the sulfides being most preferred.

The second component of the catalyst preferably consists essentially of the alkali elements or alkaline earth elements in free or combined form. Alkali elements include lithium, sodium, potassium, rubidium and cesium. Alkaline earth elements include magnesium, calcium, strontium and barium. Alkali elements and in particular cesium and potassium are preferred. Potassium is most preferred. The second component or (promoter) may be present in free or combined form as a metal, oxide, hydroxide, sulfide or as a salt or a combination of these. The alkaline promoter is preferably present at a level sufficient to render the catalyst neutral or basic.

The second component may be: added as an ingredient to the molybdenum or tungsten component; added to the support; a part of one of the other components, such as sodium or potassium molybdate; or an integral part of the support. For example, carbon supports prepared from coconut shells often contain small amounts of alkali metal oxides or hydroxides; or the support may contain a substantial amount of the promoter, such as when the support is magnesia.

The third optional component of the catalyst preferably consists essentially of iron, cobalt or nickel or a mixture thereof in free or combined form. Cobalt and nickel are preferred. Representative compounds include the sulfides, carbides, oxides, halides, nitrides, borides, salicylides, oxyhalides, carboxylates such as acetates, acetylacetonates, oxalates, carbonyls and the like. Representative compounds also include the elements combined with first component elements in the anionic form such as iron, cobalt or nickel molybdates, phosphomolybdates, tungstates, phosphotungstates and the like. The sulfides, carbonyls, carbides and oxides are preferred with the sulfides being most preferred.

A fourth optional component of the catalyst is a support which can assume any physical form such as a powder, pellets, granules, beads, extrudates, or others. The supports may be coprecipitated with the active metal species. The support in powder form may be treated with an active metal species and then used or formed into the aforementioned shapes. The support may be formed into the aforementioned shapes and then treated with the active catalytic species.

The first two or three components may be dispersed on the support by methods known in the art. Examples include: impregnation from solution which may be followed by conversion to the active species, vapor deposition, intimate physical mixing, sulfiding of other first or second component species, precipitation of sulfides in the presence of the support and the like. One or more of these methods may be used.

Exemplary support materials include: the aluminas, basic oxides, silicas, carbons or suitable solid compounds of magnesium, calcium, strontium, barium, scandium, yttrium, lanthanum and the rare earths, titanium, zirconium, hafnium, vanadium, niobium, tantalum, thorium, uranium and zinc. Oxides are exemplary compounds. Preferably, the supports are neutral or basic or may be rendered neutral or basic by addition of the alkaline promoters. The aluminas include the alpha, gamma and eta types. The silicas include for example silica gel, diatomaceous earth and crystalline silicates.

The carbon supports, which are preferred, include activated carbons such as those prepared from coals and coal-like materials, petroleum-derived carbons and animal- and vegetable-derived carbons. Preferably, the carbon support will have a surface area of 1 to 1500 $m^2/g$, more preferably 10 to 1000 $m^2/g$ and most preferably 100 to 500 $m^2/g$, as measured by the BET nitrogen test. Exemplary carbon supports include coconut shell charcoal, coals, petroleum cokes, carbons formed by pyrolyzing materials such as vinylidene chloride polymer beads, coal, petroleum coke, lignite, bones, wood, lignin, nut shells, petroleum residues, charcoals, and others.

For several reasons, the preferred form of the catalyst is the alkalized agglomerated sulfide. Certain forms of cobalt/molybdenum sulfide are more preferred. Most preferred is agglomerated, cobalt/molybdenum sulfide in which the cobalt and molybdenum sulfides are coprecipitated.

Methods for making sulfide catalysts are disclosed generally at pages 23—34 of *Sulfide Catalysts, Their Properties and Applications*, O. Weisser and S. Landa, Pergamon Press, New York, 1973. Sulfide catalysts may be made by precipitating iron, cobalt or nickel sulfide in the presence of ammonium tetrathiomolybdate or other thiomolybdates or thiotungstates and thereafter thermally treating the mixture to convert the thiomolybdate or thiotungstate salt to the sulfide. Alternatively, one may use the methods disclosed in U.S. Patents 4,243,553 and 4,243,554. Combined first and third component sulfides are available commercially from several catalyst manufacturers.

Cobalt and molybdenum may be impregnated as salts on a support, then calcined to the oxide and then sulfided with hydrogen sulfide as taught in G.B. patent publication 2,065,491. A cobalt/molybdenum sulfide may be precipitated directly onto a support, but the unsupported cobalt/molybdenum sulfide is preferred. Other combinations of first and second component sulfides may be similarly made.

An unsupported catalyst preferably has a surface area of at least about 10 $m^2/g$ and more preferably

more than 20 $m^2/g$ as measured by the BET nitrogen surface area test.

The preferred method of making a cobalt/molybdenum sulfide or other first and third component sulfide is by adding solutions of ammonium tetrathiomolybdate or other equivalent salt and a cobalt or nickel salt such as the acetate more or less simultaneously to 30 percent acetic acid. This results in a coprecipitation of cobalt or nickel/molybdenum sulfide. By varying the ratios of cobalt or nickel and molybdenum or other salts in the solutions, one may vary the ratio of cobalt or nickel and molybdenum or other elements in the sulfide catalyst.

The cobalt/molybdenum sulfide or other sulfide may then be separated from the solvent, dried, calcined and blended with a second component promoter such as potassium carbonate and agglomerating agents and/or pelleting lubricants, then pelleted and used as a catalyst in the process. It is preferred to protect such catalysts from oxygen from the time it is made until it is used.

The alkali or alkaline earth second component may be added to the active catalytic elements prior to, during or after the formation of the sulfide by physical mixing or solution impregnation. The active metal sulfide may then be combined with binders such as bentonite clay and/or pelleting lubricants such as Sterotex® and formed into shapes for use as a catalyst.

The finished catalyst may be used in a fixed bed, moving bed, fluid bed, ebullated bed or a graded fixed bed in which the concentration and/or activity in the catalyst varies from inlet to outlet in similar manner to known catalysts. The catalyst may be used in powder form or may be formed into shapes with or without a binder.

Catalysts used in the present process preferably contain less than 25 weight percent (based on the total weight of carbon oxide hydrogenation active metals) of other carbon oxide hydrogenation active metals, more preferably less than 20 weight percent, and most preferably less than 2 weight percent. The catalyst may be essentially free of other carbon oxide hydrogenating components. By "essentially free" it is meant that the other carbon oxide hydrogenating components do not significantly alter the character or quantity of the alcohol fraction. For example, a significant change would be a 5 percent change in the amount of the alcohol fraction or a 5 percent change in the percentage of any alcohol in the alcohol fraction.

Carbon oxide hydrogenating components present in thus limited quantities or excluded are preferably those that contain chromium, manganese, copper, zinc, ruthenium and rhodium. More preferably, in addition to the above-mentioned components, those that contain halogen such as iodine, rhenium, titanium, vanadium, cerium, thorium, uranium, iridium, palladium, platinum, silver and cadmium are excluded.

Preferably, compounds acting as ligands are also absent. Exemplary ligands which may be limited or excluded include those that are disclosed in U.S. Patent 4,405,815. These are generally polydentate ligands in which the donor atoms are phosphorous, arsenic, antimony or bismuth, though other monodentate and polydentate ligands may also be excluded.

It may be advantageous to use conditions for the reaction which encourage low methanol to higher alcohol ratios in the product stream. The conditions under which this reaction occurs are similar to those under which mixed alcohols may be formed directly from the $H_2/CO$ synthesis gas. Accordingly, the alcohol conversion and the alcohol synthesis reactions take place simultaneously.

In the normal operating ranges, the higher the pressure at a given temperature, the more selective the alcohols synthesis process will be to alcohols. The minimum contemplated pressure is about 500 psig (3.55 MPa). The preferred minimum is about 750 psig (5.27 MPa) with about 1000 psig (7.00 MPa) being a more preferred minimum. While about 1500 psig (10.4 MPa) to about 4000 psig (27.7 MPa) is the most desirable range, higher pressures may be used and are limited primarily by the cost of high pressure vessels and compressors needed to carry out the higher pressure reactions. The typical maximum is about 10,000 psig (69.1 MPa) with about 5000 psig (34.6 MPa) a more preferred maximum. Thus, the pressure range is from about 500 psig (3.55 MPa) to about 10,000 psig (69.1 MPa). A most preferred operating pressure is about 3000 psig (20.8 MPa).

The minimum temperature used is governed by productivity considerations of the alcohols synthesis reaction and the fact that at temperatures below about 200°C volatile metal carbonyls may form. Alcohols may also condense at lower temperatures. Accordingly, the minimum temperature is generally around 200°C. A preferred minimum temperature is 240°C. A maximum temperature is about 400°C. Thus, the temperature range is from about 200°C to about 400°C. Preferably, the maximum temperature is about 375°C or less, more preferably 350°C or less and the most preferred range is from about 240°C to about 325°C.

The $H_2/CO$ gas hourly space velocity (GHSV) is a measure of volume of the hydrogen plus carbon monoxide gas at standard temperature and pressure passing a given volume of catalyst in an hour's time. This may range from about 100 to about 20,000 $hr^{-1}$ and preferably from about 300 to about 5000 $hr^{-1}$. Selectivity to alcohols of the alcohols synthesis process generally increases as the space velocity increases. However, conversions of carbon monoxide and hydrogen decrease as space velocity increases and the selectivity to higher alcohols decreases as space velocity increases.

Preferably, at least a portion of the unconverted hydrogen and carbon monoxide in the product gas from the reaction, more preferably after removal of product alcohols, water and carbon dioxide formed and even more preferably any hydrocarbons formed, may be recycled to the reaction. One may wish to isolate one or more of the alcohols and recycle that also. That is typically done in a separate step wherein the

alcohol to be recycled is isolated from the balance of the alcohols. The amount of recycle is expressed as the recycle ratio which is the ratio of moles of gases in the recycle stream to the moles of gases in the fresh feed stream. A recycle ratio of zero is within the scope of the present process with at least some recycle preferred. A recycle ratio of at least about one is more preferred and at least about 3 is most preferred.

The rate of alcohol fed to the process is generally expressed as liquid hourly space velocity (LHSV) which is defined as the volume of liquid alcohols feed at 0°C and 760 mm Hg pressure which is vaporized and passes over a catalyst bed in an hour's time ratioed to the volume of the catalyst bed. This feed rate may range from about 0.01 $hr^{-1}$ to about 5 $hr^{-1}$ and preferably from about 0.05 $hr^{-1}$ to about 0.5 $hr^{-1}$. Preferably, there is a substantial excess of syngas to alcohol feed and generally, a ratio of at least 2 moles of hydrogen or carbon monoxide per mole of alcohol in the feed. The rate of feed of alcohol in the Examples hereinafter may be converted to LHSV by dividing the g/hr by the density of the alcohol fed (0.7931 g/cm³ for methanol) to get the volume/hr. This resultant number is then divided by the volume of the catalyst bed to yield the LHSV in units of $hr^{-1}$. As an illustration of this calculation, the data from Example 1 provided:

$$\left(\frac{5.9\text{g/hr}}{0.7931\ \text{g/cm}^3}\right) \div 30\ \text{cm}^3 = 0.248\ \text{hr}^{-1}$$

Similarly, the LHSV data for the other Examples is:

| Example No. | LHSV ($hr^{-1}$) |
|---|---|
| 2 | 0.294 |
| 3 | 0.140 |
| 4 | 0.093 |
| 5 | 0.035 |

The alcohol fraction formed boils in the motor gasoline range. The minimum boiling pure alcohol is methanol at 64.7°C. ASTM D—439 calls for a 225°C end-point for automotive gasoline. Accordingly, the alcohol preferably boils in a range from about 60°C to about 225°C when distilled by ASTM D—86. It is not necessary that the entire liquid product boil in this range, but it is preferred. It is not necessary that the alcohol fraction meet all the distillation specifications for motor gasoline; only that it boil within the broad range of motor gasolines. For example, it need not be within the 50 percent evaporated limits as set by ASTM D—439.

Because two processes are taking place simultaneously, one must look at incremental yields to see the extent to which lower alcohols are being converted to higher alcohols. One process is a Fischer-Tropsch type process wherein $H_2/CO$ are converted directly to mixed alcohols. And in fact, some of these alcohols after they are formed during the course of the process, are converted to higher alcohols within the reactor.

The second process comprises the conversion of the lower alkanols which are fed to the process into higher alcohols.

When the alcohol is easily differentiated from the products of the Fischer-Tropsch type alcohol synthesis, differentiation of the two processes is quite simple. For example, when the added alkanol is isopropanol, one need only analyze for homologues of isopropanol in the product to adequately define the yields. However, when the alcohol fed is methanol, for example, one must look at incremental yields, that is, look at the yield structure of the mixed alcohols formed without the addition of methanol and then look at the yield structure when methanol is added in addition to the $H_2/CO$ feed. Using either of these schemes, preferably at least 25 percent of the alcohol added to the reaction is converted to higher homologues.

Under preferred conditions, the amount of water formed is substantially less than the amount of alcohols formed. Typically, there is less than 20 weight percent and preferably less than 10 weight percent water based on the quantity of alcohol. This water may be removed by known techniques if the alcohol fraction is to be used as a motor fuel additive. If the water content is about 5 weight percent or less based on alcohols, the water may advantageously be removed by absorption on molecular sieves. At higher water contents one may use a water-gas shift drying step as disclosed in British patent publications 2,076,015 and 2,076,423.

The product mixture, as formed under preferred conditions, contains small portions of other oxygenated compounds besides alcohols. These other compounds may not be deleterious to use in the product in motor fuels. However, these other oxygenates, generally acetate esters, are formed in higher proportions when alcohols are added to the $H_2/CO$ feed.

Preferably, the coproducts formed with the alcohol fraction are primarily gaseous products; that is, $C_1$—$C_4$ hydrocarbons. Preferably, $C_5$ and higher hydrocarbons are coproduced at less than about 20 percent $CO_2$-free carbon selectivity, more preferably at less than 10 percent and most preferably at less than 5

percent. Lower amounts of normally liquid hydrocarbons make the normally liquid alcohols easier to separate from by-products.

The following examples are provided to illustrate the present process. Other variations and modifications are possible within the scope of this process and claims.

## EXAMPLES

### Comparison A and Example 1

Comparison A and Example 1 are conducted using a catalyst comprising molybdenum supported on carbon.

Approximately a cubic foot of Witco MBV 4—6 mesh carbon in the form of 3/16-inch (0.48 cm) extrudates is immersed for 10 minutes at 60°C—70°C in a solution containing 155.5 pounds of 22 percent ammonium sulfide, 26 pounds of ammonium heptamolybdate and 6.5 pounds of potassium carbonate. The extrudates are then removed and the excess liquor is drained therefrom. The extrudates are then calcined in nitrogen at 300°C for four hours. These steps are repeated until the extrudates have absorbed 20 percent molybdenum and 5 percent potassium based on the total weight of the catalyst. After the last impregnation, the calcination temperature is raised to 500°C. The catalyst is then passivated with 2 percent oxygen in nitrogen at a maximum temperature of 70°C.

The catalyst is placed in a ½-inch (1.27 cm) stainless steel tube. Total volume of the catalyst is 30 $cm^3$. The weight of catalyst is 22 g. Premixed carbon monoxide and nitrogen from a cylinder is passed through a molecular sieve bed at ambient temperature to remove iron and other carbonyls. Hydrogen and hydrogen sulfide from cylinders is then mixed with the carbon monoxide and nitrogen and the mixture is compressed to the pressure stated. The nitrogen is added at about a 5 percent level to serve as an internal standard for analytical purposes. Methanol is added as stated using a high pressure liquid pump.

The combined feed gas and methanol stream is then preheated and passed at the stated hourly space velocities through the fixed bed reactor which is maintained at the stated reaction temperatures by an electric furnace.

The reactor product is passed through a pressure letdown valve into a vapor-liquid separator at room temperature. The product gases leaving the separator flow past the gas chromatograph sampling port, through a second pressure letdown valve into a dry ice cooled condenser. Liquid products from the vapor-liquid separator and the condenser are collected, weighed, sampled and analyzed. The data sets given represent the combined analyses of both of these samples. The results of the experiment are shown in Table I.

TABLE I

| | Comparison A | Example 1 |
|---|---|---|
| Temperature (°C) | 260 | 260 |
| Pressure (psig) | 2500 | 2500 |
| (MPa) | 17.3 | 17.3 |
| $H_2$/CO GHSV ($hr^{-1}$) | 1870 | 1870 |
| $H_2$/CO mole ratio | 1.12 | 1.12 |
| $H_2S$ level (ppm)* | 20 | 20 |
| Methanol feed rate (g/hr) | 0 | 5.9 |
| Methanol (g/hr) | 2.42 | 7.76 |
| Ethanol | 0.81 | 1.57 |
| Propanols | 0.15 | 0.27 |
| Butanols | 0.016 | 0.023 |
| Pentanols | - | - |
| Methyl Acetate | 0.0433 | 0.211 |
| Ethyl Acetate | 0.016 | 0.015 |

*vol/vol based on $H_2$/CO

The reaction conditions for Comparison A and Example 1 are identical except that methanol is not fed during Comparison A but is fed during Example 1. The addition of methanol results in an increase in the rate of production of higher alcohols. The large increase in the production of acetate esters suggests that the reaction may proceed through the formation of these acetates.

Comparison B and Example 2

The catalyst used in this example is a coprecipitated molybdenum cobalt sulfide.

Ammonium tetrathiomolybdate $(NH_4)_2MoS_4$ and cobalt acetate, in a mole ratio of molybdenum to cobalt of 2 to 1, are added to 30 percent acetic acid at 50°C. The precipitate is filtered and dried under nitrogen at 120°C and then calcined for 1 hour at 500°C in a nitrogen atmosphere. The dry cake is then ground together with potassium carbonate, bentonite clay and Sterotex® to achieve a weight ratio of 66 weight percent $(CoS/2MoS_2)$, 10 weight percent $K_2CO_3$, 20 weight percent bentonite clay and 4 weight percent Sterotex®. This mixture is then pelleted at 30,000 psig and the pellets are stored under nitrogen until used.

The reaction system for these examples is the same as that for Comparison A and Example 1. Total volume of catalyst is 30 $cm^3$. The weight of catalyst is 32.7 g. The operating conditions and the results are shown in Table II.

TABLE II

| | Comparison B | Example 2 |
|---|---|---|
| Temperature (°C) | 290 | 290 |
| Pressure (psig) | 2000 | 2000 |
| (MPa) | 13.8 | 13.8 |
| $H_2/CO$ GHSV ($hr^{-1}$) | 2000 | 2000 |
| $H_2/CO$ mole ratio | 1.05 | 1.05 |
| $H_2S$ level (ppm)* | 30 | 30 |
| Methanol feed rate (g/hr) | 0 | 7.0 |
| Methanol (g/hr) | 1.96 | 2.73 |
| Ethanol | 3.31 | 5.36 |
| Propanols | 1.00 | 1.51 |
| Butanols | 0.24 | 0.38 |
| Pentanols | - | - |
| Methyl Acetate | 0.070 | 0.148 |
| Ethyl Acetate | 0.108 | 0.247 |

*vol/vol based on $H_2/CO$

The reaction conditions for Comparison B and Example 2 are identical except that methanol is not fed during Comparison B but is fed during Example 2. The addition of methanol results in an increase in the production of higher alcohols and again in the increase in production of acetate esters.

Comparison C and Example 3

In additional experiments ethanol is added to the $H_2/CO$ feed.

The catalysts in these two examples are the same as those used in Example 2. The reaction is carried out in the same apparatus as Example 2. The reaction parameters and results are set out in Table III.

TABLE III

| | Comparison C | Example 3 |
|---|---|---|
| Temperature (°C) | 289 | 289 |
| Pressure (psig) | 2000 | 2000 |
| (MPa) | 13.8 | 13.8 |
| $H_2/CO$ GHSV ($hr^{-1}$) | 3000 | 3000 |
| $H_2/CO$ mole ratio | 1.08 | 1.08 |
| $H_2S$ level (ppm)* | 90 | 90 |
| Ethanol feed rate (g/hr) | 0 | 3.32 |
| Methanol (g/hr) | 1.80 | 1.76 |
| Ethanol | 1.36 | 3.24 |
| Propanols | 0.344 | 0.513 |
| Butanols | 0.083 | 0.061 |
| Pentanols | 0.011 | 0.000 |
| Methyl Acetate | 0.046 | 0.055 |
| Ethyl Acetate | 0.026 | 0.092 |

*vol/vol based on $H_2/CO$

It can be seen that the ethanol is converted to higher alcohols. The conversion is not as efficient, however, as it is for methanol.

Comparison D and Example 4

Using the same catalyst and reactor setup as Example 2, the reaction with ethanol is carried out again. The reaction conditions and results are as set out in Table IV.

TABLE IV

| | Comparison D | Example 4 |
|---|---|---|
| Temperature (°C) | 270 | 270 |
| Pressure (psig) | 3000 | 3000 |
| (MPa) | 20.8 | 20.8 |
| $H_2/CO$ GHSV ($hr^{-1}$) | 2000 | 2000 |
| $H_2/CO$ mole ratio | 1.05 | 1.05 |
| $H_2S$ level (ppm)* | 30 | 25 |
| Ethanol feed rate (g/hr) | 0 | 2.21 |
| Methanol (g/hr) | 1.078 | 1.198 |
| Ethanol | 0.612 | 1.762 |
| Propanols | 0.171 | 0.244 |
| Butanols | 0.054 | 0.041 |
| Pentanols | 0.006 | 0.000 |
| Methyl Acetate | 0.064 | 0.081 |
| Ethyl Acetate | 0.027 | 0.090 |

*vol/vol based on $H_2/CO$

Again, the ethanol is apparently converted to higher alcohols.

Comparison E and Example 5

The catalyst used in this example is an unsupported molybdenum disulfide catalyst comprising pelletized 66 percent $MoS_2$ (High Surface Area Special $MoS_2$ obtained from Climax Molybdenum), 20 percent bentonite clay, 10 percent $K_2CO_3$, and 4 percent Sterotex®.

In Comparison E and Example 5, the reactor consists of a jacketed stainless steel pipe packed with catalyst. The total volume of catalyst is about one cubic foot (0.028 $m^3$). The reactor jacket carries a heat-transfer fluid to remove the heat of reaction. The carbon monoxide feed gas passes through a bed of molecular sieves at room temperature to remove iron and other carbonyls. The hydrogen and carbon monoxide feed gases are then mixed at the ratio stated with 300 ppm hydrogen sulfide. Nitrogen is added to the feed gas at two percent by volume as an internal standard and the mixture is compressed to the pressure stated. Methanol is added. The methanol and feed gas mixture is preheated to the stated reaction temperature and then passed through the fixed-bed reactor at the stated hourly space velocity. The reactor products pass through a water-cooled condenser into a high pressure vapor/liquid separator. The product liquids from the high pressure separator pass through a pressure letdown valve into a low pressure vapor/liquid separator. The product gases leaving the high pressure separator pass through a pressure letdown valve, are combined with the gases from the low pressure separator, and flow past a gas chromatograph sampling point. Liquid products from the low pressure separator are collected in a receiver where they may be sampled and analyzed.

The reactor operating conditions for Comparison E and Example 5 are shown in Table V.

TABLE V

| | Comparison E | Example 5 |
|---|---|---|
| Temperature (°C) | 233 | 233 |
| Pressure (psig) | 1800 | 1800 |
| (MPa) | 12.5 | 12.5 |
| $H_2$/CO GHSV ($hr^{-1}$) | 896 | 888 |
| $H_2$/CO mole ratio | 1.04 | 1.05 |
| $H_2S$ level (ppm)* | 300 | 300 |
| Methanol feed rate (g/hr) | 0 | 766.57 |
| Methanol (g/hr) | 683.51 | 1050.29 |
| Ethanol | 305.31 | 333.66 |
| Propanols | 43.50 | 47.58 |
| Butanols | 7.62 | 9.66 |
| Pentanols | 0 | 0 |
| Methyl Acetate | 24.31 | 51.39 |
| Ethyl Acetate | 5.85 | 6.94 |

*vol/vol based on $H_2$/CO

The addition of methanol results in an increase in the production of higher alcohols and again in an increase in production of acetate esters.

## Claims

1. A process for preparing higher alcohols under conditions where the number of carbon atoms in the product alcohols are increased compared with the number of carbon atoms in the starting alcohols which comprises reacting:

(1) hydrogen;

(2) carbon monoxide;

(3) a heterogenous catalyst having (a) a first component of molybdenum, tungsten, or a mixture thereof in free or combined form; and (b) a second component of an alkali or alkaline earth element, or a mixture thereof in free or combined form; and

(4) one ormore lower alcohols.

2. The process of Claim 1 wherein the catalyst has a third component of cobalt, nickel or iron, or a mixture thereof in free or combined form.

3. The process of Claim 1 or 2 wherein the catalyst has a further component of a support.

4. The process of Claims 1—3 wherein the first component of the catalyst is molybdenum.

5. The process of Claims 2—3 wherein the catalyst contains molybdenum sulfide and cobalt sulfide.

6. The process of Claims 1—3 wherein the fourth reactant contains methanol.

7. The process of Claim 6 wherein the fourth reactant contains methanol obtained by fractionating a mixed alcohol product containing methanol and the higher alcohols.

8. The process of Claims 1—7 wherein the hydrogen and carbon monoxide are present in a molar ratio of from about 0.7:1 to about 3:1.

9. The process of Claims 1—8 wherein the reaction is run at a temperature from about 200°C to about 400°C.

10. The process of Claims 1—9 wherein the reaction is run at a pressure from about 500 psig (3.55 MPa) to about 10,000 psig (69.1 MPa).

11. The process of Claims 1—10 wherein at least 25 weight percent of the fourth reactant alcohols is converted to higher alcohols.

12. The process of any of the preceeding claims wherein methanol is converted to ethanol and other higher alcohols.

**Patentansprüche**

1. Verfahren zur Herstellung höherer Alkohole unter Bedingungen, bei denen die Zahl der Kohlenstoffatome in den Produktalkoholen vergrößert wird im Vergleich zur Zahl der Kohlenstoffatome in den Ausgangsalkoholen, umfassend die Reaktion von:

(1) Wasserstoff,

(2) Kohlenmonoxid,

(3) einem heterogenen Katalysator mit (a) einer ersten Komponente aus Molybdän, Wolfram oder einem Gemisch davon in freier oder kombinierter Form und (b) einer zweiten Komponente aus einem Alkali- oder Erdalkalielement oder einem Gemisch davon in freier oder kombinierter Form und

(4) einem oder mehreren niederen Alkoholen.

2. Verfahren nach Anspruch 1, worin der Katalysator eine dritte Komponente aus Kobalt, Nickel oder Eisen oder ein Gemisch davon in freier oder kombinierter Form enthält.

3. Verfahren nach Anspruch 1 oder 2, worin der Katalysator eine weitere Komponente aus einem Träger enthält.

4. Verfahren nach einem der Ansprüche 1 bis 3, worin die erste Komponente des Katalysators Molybdän ist.

5. Verfahren nach Anspruch 2 oder 3, worin der Katalysator Molybdänsulfid und Kobaltsulfid enthält.

6. Verfahren nach einem der Ansprüche 1 bis 3, worin der vierte Reaktionspartner Methanol enthält.

7. Verfahren nach Anspruch 6, worin der vierte Reaktionspartner Methanol enthält, der durch Fraktionierung eines gemischten Alkoholprodukts erhalten wurde, das Methanol und die höheren Alkohole enthält.

8. Verfahren nach einem der Ansprüche 1 bis 7, worin Wasserstoff und Kohlenmonoxid in einem Molverhältnis von ungefähr 0,7:1 bis ungefähr 3:1 vorliegen.

9. Verfahren nach einem der Ansprüche 1 bis 8, worin die Reaktion bei einer Temperatur von ungefähr 200°C bis ungefähr 400°C durchgeführt wird.

10. Verfahren nach einem der Ansprüche 1 bis 9, worin die Reaktion bei einem Druck von ungefähr 500 psig (3,55 MPa) bis ungefähr 10000 psig (69,1 MPa) durchgeführt wird.

11. Verfahren nach einem der Ansprüche 1 bis 10, worin mindestens 25 Gew.-% des vierten Alkohol-Reaktionspartners zu höheren Alkoholen umgesetzt werden.

12. Verfahren nach einem der vorhergehenden Ansprüche, worin Methanol zu Ethanol und anderen höheren Alkoholen umgesetzt wird.

**Revendications**

1. Procédé de préparation d'alcools supérieurs, dans des conditions où le nombre d'atomes de carbone dans les alcools produits est augmenté par rapport au nombre d'atomes de carbone dans les alcools de départ, lequel procédé comporte le fait de faire réagir:

(1) de l'hydrogène;

(2) du monoxyde de carbone;

(3) un catalyseur hétérogène, comportant (a) un premier composant, molybdène, tungstène, ou mélange de ceux-ci, sous forme libre ou combinée; (b) un second composant, élément alcalin ou alcalino-terreux, ou mélange de ceux-ci, sous forme libre ou combinée; et

(4) un ou plusieurs alcools inférieurs.

2. Procédé conforme à la revendication 1, dans lequel le catalyseur comporte un troisième composant, cobalt, nickel ou fer, ou un mélange de ceux-ci, sous forme libre ou combinée.

3. Procédé conforme à la revendication 1 ou 2, dans lequel le catalyseur comporte un autre composant, servant de support.

4. Procédé conforme aux revendications 1 à 3, dans lequel le premier composant du catalyseur est du

molybdène.

5. Procédé conforme aux revendications 2 et 3, dans lequel le catalyseur contient du sulfure de molybdène et du sulfure de cobalt.

6. Procédé conforme aux revendications 1 à 3, dans lequel le quatrième réactif contient du méthanol.

7. Procédé conforme à la revendication 6, dans lequel le quatrième réactif contient du méthanol obtenu par fractionnement d'un produit alcool mixte contenant du méthanol et des alcools supérieurs.

8. Procédé conforme aux revendications 1 à 7, dans lequel l'hydrogène et le monoxyde de carbone sont présents en un rapport molaire valant d'environ 0,7:1 à environ 3:1.

9. Procédé conforme aux revendications 1 à 8, dans lequel la réaction est effectuée à une température d'environ 200°C à environ 400°C.

10. Procédé conforme aux revendications 1 à 9, dans lequel la réaction est effectuée sous une pression d'environ 500 psig (3,55 MPa) à environ 10 000 psig (69,1 MPa).

11. Procédé conforme aux revendications 1 à 10, dans lequel au moins 25% en poids des alcools constituant le quatrième réactif sont convertis en alcools supérieurs.

12. Procédé conforme à l'une quelconque des revendications précédentes, dans lequel du méthanol est converti en éthanol et en d'autres alcools supérieurs.